# EUROPEAN PATENT APPLICATION

(11) **EP 2 940 150 A1**
(43) Date of publication of application: **04.11.2015**
(21) Application number: 14166385.6
(22) Date of filing: 29.04.2014
(51) Int. Cl.: C12Q 1/68

(54) **Self-assembly of DNA Origami: a new diganostic tool**

(71) Applicant: Baseclick GmbH, 82327 Tutzing (DE)
(72) Inventor: Manetto, Antonio, 80686 München (DE)
(74) Representative: Weickmann & Weickmann

(57) **Abstract**

The present invention relates to methods for detecting an analyte in a sample utilizing self-assembly of nucleic acids to yield predetermined nanostructures. Further aspects of the invention relate to stabilizing nucleic acid nanostructures and to the use thereof in methods for detecting an analyte. Also provided are kits and devices for use in the described methods.

## Description

The present invention relates to methods for detecting an analyte in a sample utilizing self-assembly of nucleic acids to yield predetermined nanostructures. Further aspects of the invention relate to stabilizing nucleic acid nanostructures and to the use thereof in methods for detecting an analyte. Also provided are kits and devices for use in the described methods.

There is a great need in the art for diagnostic assays suitable to detect biomaterials such as oligonucleotides, DNA, RNA and proteins. The methodologies available today require expensive equipment and technologies and they are exclusively suited for specialized users. In some cases these methods exhibit shortcomings in terms of specificity and require an expensive multi-component assay. Therefore a novel approach to detect DNA and RNA without any specific scientific background and without a need for complex technology would be highly desirable in order to extend these kinds of diagnostics to a large variety of applications. Further, there is a need for simple methods to detect single nucleic acid molecules. It would be particularly beneficial if a detection method was available which is easy to carry out by the user, e.g. using a visual analysis method including microscopy.

A method wherein nucleic acids are not used as carriers of genetic information but because of their ability to adopt a certain shape is nucleic acid origami. Nucleic acid origami structures, also referred to as *DNA origami structures* or *DNA origami,* are two- or three-dimensional arbitrary shapes formed from nucleic acids. The term *"origami"* infers that one or more strands or building blocks of DNA (called scaffold strands) may be folded or otherwise positioned into a desired structure or shape. The desired structure or shape which may then be secured into a desired shape or structure by one or more other strands or building blocks of DNA such as a plurality of staple strands of DNA. Methods of DNA origami are described for example by Rothemund, "Folding DNA to create nano-scale shapes and patterns", Nature, March 2006, pp. 297-302, vol. 440; Douglas et al., Nature, 459, pp. 414-418 (2009); and Seeman, "Nanomaterials based on NA", An. Ref. Biochem. 79, pp.65-87 (2010), all of which are incorporated herein by reference in their entirety.

A nucleic acid origami structure need not be constructed of a scaffold strand and staple strands but can be constructed by single-stranded nucleic acid sequences which self-assemble into tiles to form lattices of any desired shape or size. Such approaches include programmed self-assembly of designed strands of nucleic acids to create a wide range of structures with designed shapes, cf. Wei et al., Nature, vol. 485, pp. 623-627 (2012), herein incorporated by reference in its entirety.

A DNA nanostructure may be of any arbitrary shape as desired. If the nanostructure is of sufficient size, it is directly visually distinguishable from other structures. The fact whether or not a visual proof of a nucleic acid nanostructure is successful however depends on the stability of the structure. Specifically nanostructures consisting of short nucleic acid strands display a limited stability. It is therefore desirable to have ways to stabilize nanostructures.

The inventors of the the present application now found that self-assembly of nucleic acids resulting in a distinctive nanostructure can be utilized as a novel diagnostic tool. In particular, it was found that the formation of a visibly detectable nanostructure in the presence of a certain analyte can be used in a method for detecting the analyte in a sample.

Thus, a first aspect of the invention relates to a method for detecting an analyte in a sample comprising
(i) contacting the sample with a plurality of staple strands
(ii) providing conditions suitable to induce self-assembly of the staple strands and the analyte to yield a nanostructure and
(iii)determining if a predetermined nanostructure is formed, wherein the formation of the predetermined nanostructure indicates the presence of the analyte in the sample.

As used herein, the term *"nanostructure"* is defined to mean any structure formed from a plurality of elements having a distinct shape. For example, the shape may include linear forms, circular forms, two-dimensional patterns or three-dimensional structures. Preferably, at least one dimension of the structure is on the nanoscale, i.e. in the range between 0.1 and 100 nm. For example, two-dimensional patterns may have a thickness on the nanoscale. Nanotubes preferably have two dimensions on the nanoscale, i.e. the diameter of the tube is between 0.1 and 100 nm while the length could be much greater.

The number of individual elements that make up the nanostructure may be in the range from two or three up to several hundreds or even thousands of elements. Typically, a nanostructure is made from ten to 500, 50-300, e.g. 100-20 elements.

In the method of the invention, one or more copies of the analyte and a plurality of staple strands self-assemble to yield a nanostructure. According to a preferred embodiment of the invention, one copy of the analyte and a plurality of staple strands form the nanostructure. According to this aspect of the invention, it is possible to detect even single copies of an analyte present in a sample.

The term *"plurality"* as used herein is defined to mean any number of two or more, for example in the range between two and 2000, preferably 50-1000, 100-500 or 200-300. A plurality of staple strands may include one copy of each staple strand. However it is also possible that several copies of the same staple strand are present. An exemplary set of staple strands comprises two or more copies of each staple strand.

The term *"staple strand"* refers to any at least partially single-stranded nucleic acid or nucleic acid binding molecule. Examples of staple strands include DNA, RNA and nucleic acid analogues such as peptide nucleic acids (PNA), morpholino, locked nucleic acids (LNA) as well as glycol nucleic acids (GNA) and threose nucleic acids (TNA).

Individual staple strands may range in length from ten to 100, preferably from 18-50 nucleotides. The lower limit is justified by considering that the binding of shorter staples may not be stable. Cost considerations regarding a high throughput synthesis of sufficiently pure staples typically set the upper length limit.

According to a preferred aspect of the invention, extended staple strands are used which include a domain having a sequence that does not hybridize to other staple strands or to the analyte. Additional elements can be directly or indirectly attached to such staples. As used herein, the term *"directly bound"* refers to a covalent attachment while the term *"indirectly bound"* in contrast refers to the attachment to an entity through one or more non-covalent interactions.

The terms *"nucleic acid molecule", "oligonucleotide"* and *"polynucleotide"* are used herein interchangeably and are intended to include, but are not limited to, a polymeric form of nucleotides that may have various lengths, either deoxyribonucleotides or ribonucleotides or analogues thereof. Different polynucleotides may have different three-dimensional structures and may perform various functions, known or unknown. Non-limiting examples of polynucleotides include a gene, a gene fragment, an exon, an intron, intergenic DNA (including, without limitation, heterochromatic DNA), messenger RNA (mRNA), transfer RNA, ribosomal RNA, ribozymes, small interfering RNA (siRNA), cDNA, recobinant polynucleotides, branched polynucleotides, plasmids, vectors, isolated DNA of a sequence, isolated RNA of a sequence, nucleic acid probes and primers. Nucleic acids and nucleic acid-binding molecules useful in the methods described herein may comprise natural nucleic acids, sequences and variants thereof, artificial nucleic acid sequences or a combination of such sequences.

A nucleic acid molecule is typically composed of a specific sequence of four nucleotide bases adenine, cytosine, guanine and thymine (or uracil for thymine when the polynucleotide is RNA). Optionally, nucleic acid molecules may include one or more non-standard nucleotides, nucleotide analogues and/or modified nucleotides. Such molecules are included in the definition of *"nucleic acid-binding molecules".*

Examples of modified nucleotides include, but are not limited to, diaminopurine, S² T, 5-fluorouracil, 5-bromouracil, 5-chlorouracil, 5-iodouracil, hypoxantine, xantine, 4-acetylcytosine, 5-(carboxyhydroxylmethyl) uracil, 5-carboxymethylaminomethyl-2-thiouridine, 5- carboxy methyl amino methyl uracil, dihydro uracil, β-D-galactosyl guenosine, inosine, N6-isopentyl adenine, 1-methyl guanine, 1-methyl inosine, 2,2-dimethyl guanine, 2-methyl adenine, 2-methyl guanine, 3-methyl cytosine, 5-methyl cytosine, N6-adenine, 7-methyl guanine, 5-methyl amino methyl uracil, 5-methoxy amino methyl-2-thio uracil, β-D-mannosyl quenosine, 5'-metoxy carboxy methyl uracil, 5-methoxy uracil, 2-methylthio-D46-isopentyl adenine, uracil-5 oxy acetic acid, wybutoxosine, pseudouracil, quenosine, 2-thiocytosine, 5-methyl-2-thiouracil, 2-thiouracil, 4-thiouracil, 5-methyluracil, uracil-5-oxy acetic acid methyl ester, uracil-5-oxy acetic acid, 5-methyl-2-thio uracil, 3-(3-amino-3-N-2-carboxy propyl) uracil, (acp3)w, 2,6-diamino purine and the like. Nucleic acid molecules may also be modified at the base moeity (e.g. at one or more atoms that are typically available to form a hydrogen bond with a complementary nucleotide and/or at one or more atoms that are not typically capable of forming a hydrogen bond with a complementary nucleotide), sugar moiety or phosphate backbone. According to a particularly preferred embodiment of the invention, nucleic acid molecules may be modified via click chemistry wherein a desired moiety can be linked to a nucleic acid by reacting a click modified nucleic acid with a desired click modified moiety. In doing so, one of the two reaction partners carries a click reactive unsaturated group such as an alkyne group while the other reaction partner carries a click reactive 1,3-dipolar group such as an azide group. As an alternative, the modification with a desired group via click chemistry can be carried out already when producing the nucleic acid, e.g. using a click modified nucleic acid building block for the build-up of the nucleic acid. Nucleic acid molecules may also contain amine modified groups such as amino allyl-dUTP (aa-dUTP) and amino hexyl acryl amide-dCTP(aha-dCTP) to allow covalent attachment of amine reachtive moieties such as N-hydroxy succinimide esters.

Nucleic acids may be isolated from natural sources or purchased from commercial sources. In certain exemplary embodiments, nucleic acids or nucleic acid-binding molecules may be prepared using one or more of the phosphor amidite linkers and/or sequencing by ligation methods known to those of skill in the art. Oligonucleotide sequences may also be prepared by any suitable method, e.g. standard phosphor amidite methods such as those described by Bocage and Carruthirs (1981 Tetra Hedron Let. 22: 1859) or the tri-ester method according to Matteucci et al. (1981) J. Am. Chem. Soc. 103: 3185) or by any other chemical method using either a commercial automated oligonucleotide synthesizer or high-throughput, high-density array methods known in the art. Pre-synthesized oligonucleotides may also be obtained commercially from a variety of vendors.

In certain exemplary embodiments, nucleic acids or nucleic acid-binding molecules may be prepared using a variety of micro-array technologies known in the art. Pre-synthesized nucleic acids or nucleic acid-binding molecules may be attached to a support or synthesized in situ using light-directed methods, flow channel and spotting methods, ink-jet methods, pin-based methods and bead-based methods known in the art.

The analyte to be detected using the method of the invention may be any nucleic acid or nucleic acid binding molecule. The analyte may be single-stranded or double-stranded. In case of double-stranded analytes, methods for detecting same may include a denaturation step wherein the double-stranded analyte is at least partially split into single strands or single-stranded portions.

The length of the analyte molecule may range from about ten to several thousand nucleotides. According to a preferred aspect of the invention, the analyte is about 1000-3000 nucleotides (or nucleotide analog building blocks) long, e.g. 1500-2500. According to another preferred embodiment of the invention, the analyte is a shorter molecule having a length of about ten to 100 nucleotides (or nucleotide analog building blocks), e.g. 18-50.

The analyte to be detected using the method of the invention is preferably selected from analytes present in a biological or environmental sample. In particular examples of nucleic acid analytes are genomic DNA, mRNA or products derived therefrom, e.g. cDNA.

Due to its high sensitivity, the method of the present invention is suitable for detecting analytes directly without amplification. Even minute amounts of analytes, for instance of nucleic acids, e.g. 0.1 ng or lower, preferably 0.01 ng or lower, more preferably 1 pg or lower, still more preferably 0.1 pg or lower, even more preferably 0.01 pg or lower and most preferably 0.001 pg or lower may be determined without amplification.

The high sensitivity of the method of the present invention allows for the detection of analytes in the picomolar range and it is even possible to detect single molecules of the analyte.

The sample may be any sample which may contain the analyte to be detected. For example, the sample may be a biological sample such as an agricultural sample, e.g. a sample comprising plant material and/or material associated with the site where plants grow, plant materials are stored or processed. Also, the sample may be a clinical sample, such as a tissue sample or a body fluid sample such as blood, serum, plasma etc., particularly of human origin. Further types of samples include, but are not limited to, environmental samples, soil samples, food samples, forensic samples or samples from valuable goods which are tested for brand protection.

Step (i) of the method of the invention involves contacting the sample with a plurality of staple strands. If the analyte to be detected in the sample is double-stranded, step (i) may comprise establishing conditions wherein the double-stranded analyte is at least partially split into single strands or single-stranded portions. This can for example be achieved by raising the temperature. Suitable conditions are described in the art.

In one aspect of the invention, the analyte is a long scaffolding strand which binds with the plurality of staple strands that lock it into its final structure. This means the staple strands are used to fold the analyte strand into a well-defined shape. The resulting nanostructure may be designed to have an arbitrary shape. Depending on which analyte is to be detected, specific staple strands are selected capable of binding the analyte concerned.

Binding of analytes and staple strands preferably involves hybridization of complementary strands establishing a non-covalent sequence-specific interaction between analyte and staple strands. Suitable conditions where hybridization occurs are described in the art.

The staple strands are designed in a way that when binding to the analyte a predetermined nanostructure is obtained. The nanostructure may have an arbitrary shape including linear forms, circular forms, 2D and 3D forms etc.

In step (iii) of the method of the invention it is determined if a predetermined nanostructure has been formed.

According to one embodiment of the invention, the nanostructure resulting from self-assembly of the staple strands and the analyte is detected using optical methods. For example, the formation of a predetermined nanostructure can be detected using microscopic methods such as electron microscopy, atomic force microscopy and fluorescence microscopy. Other optical methods suitable for detecting if a predetermined nanostructure has been formed include laser scanners, pen type readers, CCD readers and other bar code reader or bar code scanner methods. Further, the detection may involve the use of mass spectroscopy or gel electrophoresis.

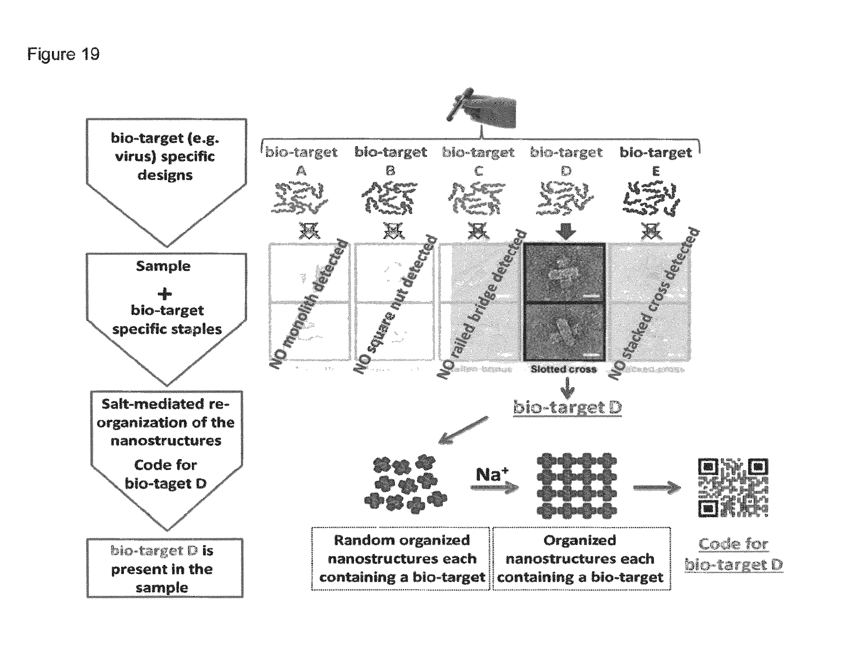

If a predetermined nanostructure is extremely small or has a less characteristic shape, nanostructure detection can be enhanced by inducing the organization of several nanostructures to form a higher-level structure. For example, the addition of a salt (e.g. monovalent Na⁺) induces a spatial re-organization of nanostructures on a solid support due to the floating on the surface effect and to the tendency to minimize the energy among the nanostructures on a surface. For example, slotted cross nanostructures can be visualized on a solid support (e.g. mica) in a low/random organized network of slotted crosses (cf. Figure 19). By the addition of a certain concentration of Na⁺ the slotted crosses move in a more organized and large network of slotted crosses. This effect can help the visualization of the network (and thus the visualization of the analyte as a macro-structure) using just low-resolution microscopes or optical instruments such as those used in bar code scanners.

Moreover, this higher-level structure can be *"blocked"*by fixing the position of the individual nanostructures, e.g. the pattern of slotted crosses. This can for example be done by means of click chemistry (e.g. when each side of the slotted crosses has click reactive groups) or via any other cross-linking chemical or enzymatical reaction. According to this aspect of the invention, the components of the predetermined nanostructure are selected in a way to ensure that functional groups, e.g. click reactive groups, are located on one or several outsides of the nanostructure which can then be specifically reacted with functional groups of other such nanostructures. In a particularly preferred embodiment, one predetermined nanostructure intended for this purpose comprises at least one click reactive unsaturated group (e.g. alkyne group) and at least one click reactive 1,3-dipolar group (e.g. azide group). When reacting the click reactive groups, the nanostructures are linked with each other so that the higher-level structure obtained by way of re-organization is fixed.

According to another embodiment of the invention, the nanostructure resulting from self-assembly of the staple strands and the analyte is indirectly determined by detecting fragments thereof. According to this aspect the method of the invention involves
(a) providing conditions wherein a nanostructure resulting from step (ii) is at least partially dissolved to yield fragments and
(b) detecting fragments of the predetermined nanostructure.

This embodiment of the present invention is particularly suitable when the originally resulting nanostructure displays a limited stability and is prone to dissolve. In this event it is reasonable to first provide specific conditions within the framework of step (a) leading to a dissolution or at least partial dissolution of the original nanostructure and to detect characteristic fragments of the nanostructure thereafter.

In one embodiment, it is for instance possible to pre-organize specific sections of analyte and/or staple strand by way of self-assembly of analyte and staple strand so that they come in close vicinity and can react with each other. It is particularly advantageous when a linkage is formed by this method which remains intact even when the nanostructure is dissolved at a later point in time.

According to this aspect of the invention it is preferred if at least one of the staple strands comprises a first functional group and at least one staple strand comprises a second functional group capable of reacting with the first functional group. If, in the presence of the analyte, a nanostructure is formed wherein the first and the second functional groups are pre-organized to come in close vicinity, the first and the second functional groups can react with each other to form a linkage. Preferably, the linkage is sufficiently stable and withstands conditions wherein the originally formed nanostructure is dissolved. When a linkage can be observed, this means that a certain nanostructure had been established even if this is not detectable any longer under the detection conditions.

The first and the second functional groups may be present on different staple strands. Additionally and/or alternatively, at least one staple strand may comprise both a first and a second functional group. Moreover, it is possible to include further functional groups for forming different additional linkages.

According to a preferred embodiment of the present invention, the first and the second functional groups may be click functional groups.

The click reaction is hereinafter intended as a reaction between a 1,3-dipolar moiety which is called a click reactive 1,3-dipolar group with an unsaturated moiety which is called a click reactive unsaturated group. The click reaction may be catalyzed by a hydrogenous catalyst, e.g. a hydrogenous copper (I) or other metal catalyst. Preferably, the click reaction comprises a (3+2) 1,3-dipolar cycloaddition reaction resulting in a 5-membered heterocyclic moiety, such as 1,2,3-triazole moiety.

Preferred examples of click reactive unsaturated groups are dipolarophiles such as alkenes and alkynes and molecules that possess related heteroatom functional groups (such as carbonyls and nitriles). Especially preferred examples of click reactive unsaturated groups are alkynes.

Preferred examples of click reactive 1,3-dipolar groups are compounds containing one or more heteroatoms which can be described as having at least one mesomeric structure that represents a charged dipole. Preferred are linear 1,3-dipolar groups, e.g. propargyl-allenyl-type diols or acides.

In addition, any other combination of first and second functional groups is possible provided that the reaction of the functional groups results in a linkage.

Examples of functional groups suitable for use in the method of the present invention are alkenyl, alkynyl, phenyl, benzyl, halo, hydroxy, carbonyl, aldehyde, haloformyl, carboxylate, carboxyl, ester, methoxy, hydroperoxy, peroxy, ether, hemiacetal, hemiketal, acetal, carboxamide, amines, imines, imide, acide, azo, cyanate, isocyanate, nitrate, nitrile, isonitrile, nitroso oxy, nitro, nitroso, pyridyl, thiol, sulfide, disulfide, sulfinyl, sulfonyl, sulfino, sulfo, thiocyanate, isothiocyanate, phosphino, phosphono, phosphate, etc.

According to one embodiment of the invention, the linkage resulting from the reaction of the first and the second functional groups stabilizes the nanostructure. According to this aspect, the stabilized nanostructure remains intact even under conditions where a nanostructure without a linkage would at least partially dissolve.

According to another embodiment of the invention, a reaction of the first and the second functional groups results in a linkage wherein one or more of the staple strands and/or the analyte are linked to form a characteristic substructure. For example, linkage of the first and the second functional groups may result in the formation of a closed strand or ring structure. The terms *"closed strand"* and *"ring structure"* are herein used interchangeably and refer to any closed structure formed from one or more staple strands and optionally the analyte.

The nanostructure formed by self-assembly of the staple strands and the analyte is visually recognizable and therefore distinguishable from other unique nanostructure shapes. Methods of making unique DNA nanostructures of arbitrary or desired design are described in Rothemund, Folding DNA to create nanoscale shapes and patterns, Nature 2006, page 297-302, Viol. 420; Rothemund, Design of DNA origami, Proceedings of the International Conference of Computer-aided Design (ICCAD) 2005 and US 7,842,792, each of which are herein incorporated by reference in their entirety.

According to an additional embodiment, a nanostructure may include one or more detectable moieties at one or more locations within or on the nanostructure. According to an exemplary embodiment, a nanostructure may be encoded with features specific to a particular analyte. Additionally, the nanostructure may be tagged with metal nanoparticles or fluorophores to enhance the distinguishability when analyzed or imaged. Additionally, the nanostructure may be tagged with metal nanoparticles or fluorophores at distinct locations to enhance distinguishability when analyzed or imaged.

According to one aspect, a scanning instrument can be used to visualize and distinguish nucleic acid nanostructures. In an exemplary embodiment, the scanning instrument is an electron microscope. Exemplary electron microscopes include a scanning electron microscope (SEM), a scanning transition electron microscope (STM), a transition electron microscope (TEM), an environmental scanning electron microscope (ESEM), a cryo electron microscope (cryo-EM) and other electron microscopes known in the art.

According to one aspect of the invention, the electron microscope scanning system scans along the nanostructure. Image processing, edge detection and object recognition algorithms can be used to detect the end points and direction vector of the nucleic acid template and inform the motion of the stage.

According to another aspect of the invention, the nanostructure may be analyzed using e.g. a high spatial resolution microscope or super-resolution microscope such as stochastic optical reconstruction microscopy (STORM). Other stochastic methods include spectral precision distance microscopy (SPDM) and photoactivated localization microscopy (PALM). Further methods include deterministic methods such as similated emission depletion (STED), ground state depletion (GSD) and spatially structured illumination microscopy (SSIM). Still additional methods include scanning probe microscopy such as atomic force microscopy or scanning tunneling microscopy (STM), as well as magnetic particles and a magnetic pick-up similar to a hard disc drive head.

In certain embodiments of the invention, staple strands and analyte are self-assembled on a solid support. According to this aspect of the invention it is preferred that at least one staple strand is immobilized on the solid support or comprises a linker group for immobilizing the staple strand to a solid support.

Suitable supports include, but are not limited to, slides, beads, chips, particles, strands, gels, sheets, tubing, spheres, containers, capillaries, pads, slices, films, plates and the like. In various embodiments, a solid support may be biological, non-biological, organic, inorganic or any combination thereof. When using a support that is substantially planar, the support may be physically separated into regions, e.g. with trenches, grooves, wells or chemical barriers (e.g. hydrophobic coatings etc.).

In certain exemplary embodiments, the support is a micro-array. As used herein, the term *"micro-array"* refers in one embodiment to a type of assay that comprises a solid phase having a substantially planar surface on which there is an array of spatially defined, non-overlapping regions or sites. One or more of these regions or sites may contain an immobilized staple strand. According to a preferred aspect of this embodiment, each of the regions or sites contains an immobilized staple strand. The immobilized staple strands may be the same or different and may be specific for the same or different analytes.

*"Substantially planar"* means that features or objects of interest such as spatially defined regions or sites may occupy a volume that extends above or below a surface and the dimensions of which are relatively small as compared to the dimensions of the surface. For example, beads disposed on the surface of a fibre-optic bundle or oligonucleotides disposed or synthesized on a porous planar substrate create a substantially planar surface.

Spatially defined regions or sites may additionally be *"addressable"* in that their location and the identity of the immobilized staple strand at that location are known or can be determined.

Staple strands immobilized on solid supports include nucleic acids or nucleic acid binding molecules that are generated in or from an assay reaction. Typically, the staple strands on solid supports are single-stranded and are covalently attached to the solid support, usually by a 5'-end or a 3'-end. In certain exemplary embodiments, staple strands are immobilized via one or more linkers that may optionally be cleavable.

According to a particularly preferred aspect of the invention, staple strands are immobilized on solid supports via click reaction. According to this aspect of the invention, the staple strands carry a click reactive group, e.g. a click reactive unsaturated group such as an alkyne group, and the solid support carries a click reactive 1,3-dipolar group such as an azide group or vice versa.

Methods of immobilizing staple strands such as nucleic acids and nucleic acid binding molecules to a support are known in the art. As used herein, the term *"attach"* refers to both covalent interactions and non-covalent interactions. A covalent interaction is a chemical linkage between two atoms or radicals formed by the sharing of a pair of electrons (i.e. a single bond), two pairs of electrons (i.e. a double bond) or three pairs of electrons (i.e. a triple bond). Covalent interactions are also known in the art as electron pair interactions or electron pair bonds. Non-covalent interactions include, but are not limited to, van der waals interactions, hydrogen bonds, weak chemical bonds (i.e. via short-range non-covalent forces), hydrophobic interactions, ionic bonds and the like. A review of non-covalent interactions can be found in Alberts et al. in Molecular Biology of the Cell, 3rd edition, Garland Publishing, 1994.

The density of non-overlapping regions containing staple strands in a micro-array is typically greater than 100 per cm² and more typically greater than 1000 per cm². Micro-array technology relating to nucleic acids and nucleic acid binding molecules is reviewed in several references known to the skilled person.

The staple strands for use in the present invention may optionally comprise a marker group such as a detectable moiety, label or reporter. Marker groups can be attached directly or indirectly. Examples of detectable moieties include various radioactive moieties, enzymes, prostatic groups, fluorescent markers, luminescent markers, bio-luminescent markers, metal particles, protein-protein binding pairs, protein-antibody binding pairs and the like. Examples of fluorescent moieties include, but are not limited to yellow fluorescent protein, green fluorescent protein, cyan fluorescent protein, umbelliferone, fluorescein, fluorescein isothiocyanate, rhodamine, dichloro triphenylamine fluorescein, cyanine, dansylchloride, phycocyanine, phycoerythrin and the like. Examples of bio-luminescent markers include, but are not limited to, luciferase (e.g. bacterial, firefly, click-beetle and the like), luciferin, aequorin and the like. Examples of enzyme systems having visually detectable signals include, but are not limited to, galactosidases, glucorinidases, phosphatases, peroxydases, cholinesterases and the like. Identifiable markers also include radioactive compounds such as ¹²⁵I, ³⁵S, ¹⁴C or ³H. Identifiable markers are commercially available from a variety of sources.

Fluorescent labels and their attachment to nucleic acids and nucleic acid binding molecules are described in many reviews known to the skilled person. As used herein, the term *"fluorescent marker"* or *"fluorescent label"* includes a signaling moiety that conveys information through the fluorescent absorption and/or emission properties of one or more molecules. Such fluorescent properties include fluorescence intensity, fluorescence lifetime, emission spectrum characteristics, energy transfer and the like.

Commercially available marker groups may be readily incorporated into staple strands. Further, marker groups may be added during nucleic acid synthesis using for example phosphoramidite or NHS chemistry. Marker groups may be attached to the staple strands by means of click chemistry. According to this aspect of the invention, marker groups are preferably linked to the nucleic acids via a linker including an 1,2,3-triazole moiety. Protocols are known in the art for custom synthesis of nucleotides having marker groups. Nucleic acids or nucleic acid binding molecules could also be stained a priori with an interchalating dye. A plurality of marker groups for post-synthetic attachment are known.

Metallic silver or gold particles may be used to enhance the signal from fluorescently labeled staple strands.

Biotin or a derivative thereof may also be used as a label on a staple strand and subsequently bound by a detectably labeled avidin/streptavidin derivative or a detectably labeled anti-biotin antibody. Digoxigenin may be incorporated as a label and subsequently bound by a detectably labeled anti-digoxigenin antibody. In general, any member of a conjugate pair may be incorporated into a staple strand, provided that a detectably labeled conjugate partner can be bound to permit detection. As used herein, the term *"antibody"* refers to antibody molecule of any class or any subfragment thereof such as an Fab.

According to certain aspects, detectable moieties described herein are spectrally resolvable. *"Spectrally resolvable"* in reference to a plurality of fluorescent labels means that the fluorescence emission bands of the labels are sufficiently distinct, i.e. sufficiently non-overlapping, so that molecular tags to which the respective labels are attached can be distinguished on the basis of the fluorescent signal generated by the respective labels by standard photo-detection systems, e.g. employing a system of band-pass filters and photomultiplier tubes or the like.

In certain embodiments, the detectable moieties can provide higher detectability when used with an electron microscope compared with common nucleic acids or nucleic acid binding molecules. Moieties with higher detectability are often in the group of metals and organometals. While some of these moieties can readily stain nucleic acids or nucleic acid binding molecules specifically, linkers can also be used to attach these moieties to a nucleic acid or nucleic acid binding molecule. Such linkers added to nucleotides or nucleotide analogue building blocks during synthesis are acrydite and thiol modified entities, amine reactive groups and acide and alkyne groups for performing click chemistry. Some nucleic acid analogues are more detectable such as gamma adenosine triphosphate, iododeoxycytidine triphosphate and metallonucleosides in general. The modified nucleotides are added during synthesis. Synthesis may refer by example to solid support synthesis of nucleic acids or nucleic acid binding molecules.

In another embodiment of the invention, the staple strands may comprise linker groups. By means of such linker groups, the staple strands may for example be attached to a solid support.

Linkers for use in the present invention may comprise an aliphatic or cyclo-aliphatic group, an aromatic or hydro-aromatic group, an alkene group, an alkyne group and/or a polymeric group, e.g. a polyethylene gycol group. The linker may have a chain length from 1-20 or more atoms and be flexible, e.g. an alkylene-based linker, optionally containing heteroatoms such as O, S and/or N or at least partially rigid, e.g., a linker which comprises at least one rigid group selected from alkane groups, alkyne groups, cyclic groups, particularly aromatic or hydro-aromatic groups, but also cyclo-aliphatic groups and combinations thereof. According to the invention, a linker may for example contain an 1,2,3-triazole moiety.

Linker groups may also include a functional group as defined herein. For example, a linker group may include a click reactive group such as a click reactive 1,3-dipolar group (e.g. an azide group) or a click reactive unsaturated group (e.g. an alkyne group).

According to another aspect of the invention, the staple strands may comprise a protected group. For example, any of the functional groups linked to a staple strand can have a protective group. If desired, this group can be unprotected at a later point in time. According to a preferred aspect of the invention, the staple strands may comprise a protected click reactive unsaturated group such as a protected alkyne group or a protected click reactive 1,3-dipolar group such as a protected azide group.

Step (ii) of the method of the invention includes providing conditions suitable to induce self-assembly of the staple strands and the analyte. In this step, the analyte and the staple strands are hybridized. *"hybridization"* refers to the process in which single-stranded molecules (or single-stranded portions thereof) bind non-covalently to form a stable double-stranded polynucleotide. The resulting (usually) double-stranded polynucleotide is a *"hybrid"* or *"duplex".* According to the method of the invention, hybridization of staple strands and analyte leads to a nanostructure where previously single-stranded molecules or sections thereof are hybridized.

*"Hybridization conditions"* will typically include salt conditions of less than about 1 m, more usually less than about 500mM and even more usually less than about 200mM. Hybridization temperatures can be as low as 5°C but are typically greater than 22°C, more typically greater than about 30°C and often in excess of about 37°C. Hybridizations are usually performed under stringent conditions, i.e. conditions under which a staple strand hybridizes to its target substance. Stringent conditions are sequence-dependent and are different in different circumstances. Longer fragments may require higher hybridization temperatures for specific hybridization. For stringent conditions, cf. e.g. Sambrook, Fritsche and Maniatis, Molecular Cloning, A Laboratory Manual, 2nd Edition, Cold Spring Harbor Press (1989).

Another embodiment of the present invention relates to a kit for detecting an analyte. The kit comprises (i) a plurality of staple strands which are capable to self-assemble with the analyte to yield a predetermined nanostructure, and optionally (ii) a solid support.

In a preferred embodiment, at least one staple strand is immobilized on the solid support or comprises a linker group for immobilizing the staple strand on the solid support. According to a particularly preferred aspect, the solid support is a micro-array as defined above. One or more staple strands may be immobilized on spatially defined sites of the micro-array. Alternatively, one or more of the staple strands include linker groups for immobilization on a spatially defined site of the micro-array. As defined above, the staple strands may be specific for the same or different analytes.

In a preferred embodiment of the invention various different staple strands are immobilized on a micro-array which are specific for different analytes. In addition, the kit preferably comprises two or more sets of staple strands, each set specific for a different analyte. The micro-array having immobilized thereto one or more staple strands may then be contacted with the sample containing the analyte and the additional sets of staple strands. Depending on which analyte is present in the sample, the analyte hybridizes specifically to one kind of staple strand on the micro-array. On the spatially defined region of this staple strand, a nanostructure is formed by self-assembly with the analyte and the additional staple strands. This distinctive nanostructure can then be determined.

The kit as described herein is particularly suitable for use in the method of the invention for detecting an analyte in a sample.

Another embodiment of the present invention relates to a device for detecting an analyte in a sample comprising (i) a sample loading unit, (ii) a kit as described above, (iii) means for processing the sample and (iv) means for determining if a predetermined nanostructure is formed.

Means for determining if a predetermined nanostructure is formed may for example include a properly designed bar cod scanner which is capable of *"reading"* the nanostructure.

In a preferred embodiment, the methods and the reagent kits and devices of the present invention are used for agricultural applications. For example, the invention is suitable for the detection of nucleic acids or nucleic acid binding molecules from plants, plant pathogens or plant pests such as viruses, bacteria, fungi or insects. Further, the invention is suitable for detecting genetic variabilities, e.g. SNPs in plants or plant parts, plant pathogens or plant pests such as insects.

A further application is a detection or monitoring of herbicide, fungicide or pesticide resistances, tolerances or intolerances, e.g. resistances, tolerances or intolerances in fungi, insects or plants, in organisms or populations of organisms. The invention is also suitable for rapid genotyping, e.g. for the rapid detection and/or differentiation of species or strains of fungi, insects or plants. Further, detection and/or differentiation of genetically modified organisms or strains, e.g. organisms or strains of fungi, insects or plants is possible.

Further, the invention is suitable for medical, diagnostic and forensic applications, e.g. in human or veterinary medicine, e.g. for the detection of nucleic acids from pathogens, e.g. human pathogens or pathogens of livestock or pet animals.

Further preferred applications include the detection of genetic variabilities, e.g. SNPs in humans or the detection of medicament resistances, tolerances or intolerances or allergies. Further, the invention is suitable for genotyping, particularly genotyping of humans in order to determine mutations associated with pre-disposition or enhanced risk of disorders, allergies and intolerances. The invention may also be used for the detection of genetically modified organisms or strains, e.g. of bacteria or viruses but also genetically modified livestock animals etc. The invention is particularly suitable for the rapid diagnosis of diseases, e.g. genetic diseases, allergic diseases, autoimmune diseases or infectious diseases.

Furthermore, the invention is suitable for detecting the function and/or expression of genes, e.g. for research purposes.

Still a further embodiment is the use of the method for brand protection, e.g. for detecting specific information encoded in products such as valuable goods like plant protection products, pharmaceuticals, cosmetics and fine chemicals (such as vitamins and amino acids) and beverage products, fuel products, e.g. gasoline and diesel, consumer electronic appliances can be marked. Further, packaging of these and other products can be marked. The information is encoded by nucleic acids or nucleic acid binding molecules which have been incorporated into the product and/or into the packaging of a product. The information may relate to the identity of the manufacturer, to production sites, the date of production and/or distributor. By means of the present invention, rapid detection of product-specific data can be carried out.

The invention is also suitable for the field of nutrients. For example, in the feed area, animal nutrients, e.g. corn, are supplemented with a greater quantity of preservatives such as propionic acids. By applying the method of the invention, the addition of preservatives can be reduced. Further, genomic analyses using the method of the invention allow the prediciton of an individual's capability to utilize specific nutrients (nutri-genomics).

Still a further preferred embodiment refers to the field of epi-genetics. This embodiment particularly refers to an analysis of DNA, e.g. genomic DNA with regard to methylation of cytosine bases. In this embodiment, the DNA may be treated with a cytosine-specific reagent, e.g. hydracine and/or hydroxylamine. By means of the treatment, a selective reaction of either cytosine or methyl cytosine residues occurs. For example, treatment with hydroxyl amine leads to a selective modification of cytosine residues. Preferably, the reagent is added in a stoichiometric amount in order to obtain a partial modification of e.g. cytosine residues. Subsequently, the treated DNA is analysed using the method described herein above.

Another embodiment of the present invention refers to method for providing a stabilized nucleic acid nanostructure, comprising the steps
(i) providing a plurality of staple strands, wherein at least one strand comprises a first functional group and at least one strand comprises a second functional group capable of reacting with the first functional group,
(ii) providing conditions suitable to induce self-assembly of the staple strands to yield a predetermined nanostructure, wherein the first functional group and the second functional group are pre-organized to come in close vicinity, and
(iii) reacting the first and the second functional groups to yield a linkage.

The staple strands are as defined herein above. According to a preferred aspect, at least one of the staple strands is a longer nucleic acid or a nucleic acid binding molecule having a length of e.g. 1000-3000 nucleotides or nucleotide analog building blocks. Such longer staple strands are called herein "*scaffold strands*". The plurality of staple strands may for example include one or two scaffold strands and the remainder being shorter staple strands having a length of e.g. 10-100 nucleotides (or nucleotide analog building blocks).

The functional groups are as defined herein above. According to a particularly preferred aspect, the functional groups are click functional groups.

The first and the second functional groups may be present on the same or different strands. For example, one or more of the staple strands may comprise a first and/or a second functional group. If the plurality of staple strands comprises one or more scaffold strands, the functional groups may be present on the scaffold strand and/or on the shorter staple strands.

The functional groups are preferably provided on staple strands which are arranged at a position in the pre-determined nanostructure which is important for the stability. For example, functional groups can be provided in the corners of the nanostructure. What is most important however is to select the position of the functional groups so as to ensure that they are preorganized during self-assembly of staple strands in a way that they get close to each other and can react with each other.

Reacting the first and the second functional groups yields a linkage, preferably a covalent linkage.

The above-described stabilization of nucleic acid nanostructures can be applied for any method using nucleic acid nanostructures. For example, stabilized nucleic acid nanostructures are suitable for use as delivery vehicles (e.g. drug delivery vessel), diagnostic tools, etc.

Subject-matter of the invention is also a stabilized nucleic acid nanostructure that can be obtained by the above method for stabilizing a nucleic acid nanostructure. The stabilized nucleic acid nanostructure comprises a plurality of staple strands and at least one covalent linkage including a 1,2,3-triazole moiety. The linkage may be between a first staple strand and a second staple strand and/or between a first end of a staple strand and a second end of that staple strand.

Another embodiment of the invention is a kit for providing a stabilized nucleic acid nanostructure comprising a plurality of staple strands, wherein at least one strand comprises a first functional group and at least one strand comprises a second functional group capable of reacting with the first functional group to form a linkage, wherein the staple strands are capable to self-assemble, optionally together with a target strand, to yield a predetermined nanostructure.

A further embodiment of the present invention relates to an improved in-situ hybridization method utilizing self-assembly of labeled staples in the presence of an analyte. According to this aspect of the invention, a sample is contacted with a plurality of staple strands, wherein one or more staple strands comprise a marker group such as a detectable moiety. Examples for detectable moieties are described herein above. Subsequently, conditions suitable to induce self-assembly of the staple strands and the analyte are provided to yield a nanostructure having incorporated one or more detectable moieties. Using these detectable moieties the nanostructure or fragments of the nanostructure can be easily detected. In fact, the concentration of detectable moieties can be very high if each staple forming part of the nanostructure is labeled with one or more detectable moieties.

In a particularly preferred embodiment of this aspect of the invention, the detectable moieties comprise fluorescent labels. According to this aspect, the method of the invention can be used as an ultra-sensitive fluorescent in-situ hybridization (FISH) technique. The advantage of the approach according to the invention as compared with conventional FISH probes is that a high concentration of fluorescent labels can be provided and that even after repetitive washing steps there is still a sufficient amount of fluorescent labels connected with the analyte to be detected.

The invention shall be further illustrated by the following figures and examples.
**Figure 1** illustrates the method of the invention for detecting an analyte (biotarget) in a sample. Visual inspection of the nanostructure formed by self-assembly of staple strands and analyte reveals that biotarget D but none of biotargets A, B, C and E is present in the sample.

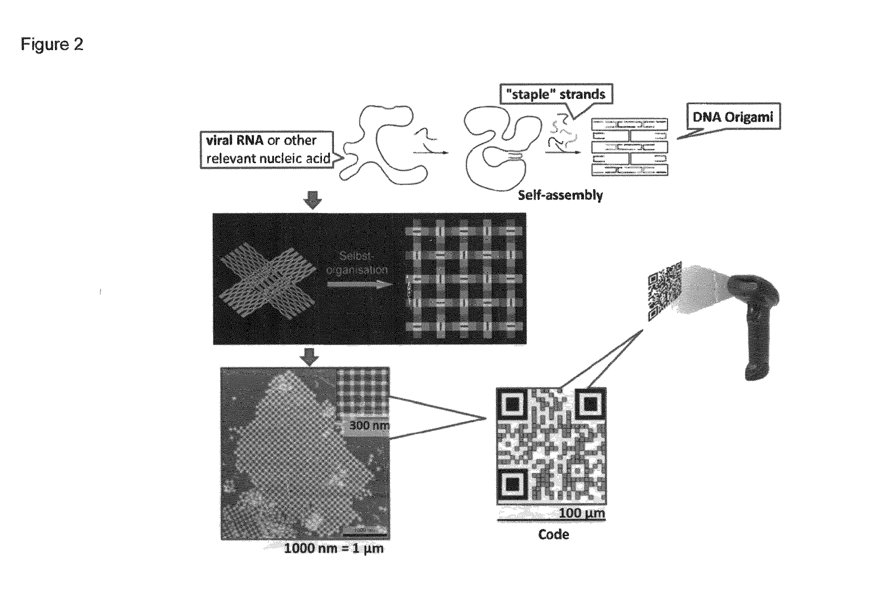

**Figure 2** illustrates the detection of an analyte using a solid support having immobilized a plurality of staple strands with specificity for different analytes. Each of the staple strands is immobilized at a predetermined position on the support. In the presence of an analyte and additional staple strands, self-assembly takes place in the region of the staple strand characteristic for the analyte. In positions where a staple strand is immobilized which does not match the analyte in the sample, no self-assembly will occur so that no nanostructure is formed in this position. The result of the assay can then be detected using e.g. a bar code scanner.
**Figure 3** illustrates an assay for detecting single nucleotide polymorphisms (SNPs). Depending on which SNP is present, a nanostructure is formed at a predefined position of a solid support. The result can be detected using a bar code scanner.

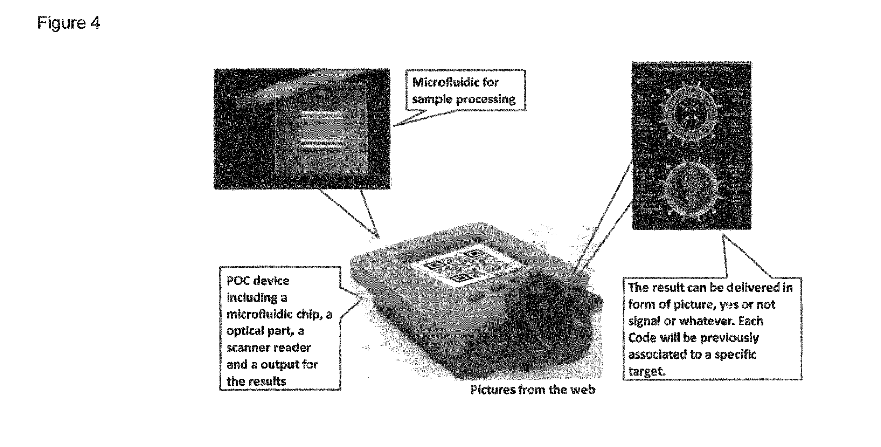

**Figure 4** shows the design of a device for detecting an analyte based on the formation of a characteristic nanostructure that can be visibly detected. The device includes a sample loading unit, a processing part, a microscope and a bar code reader/scanner.

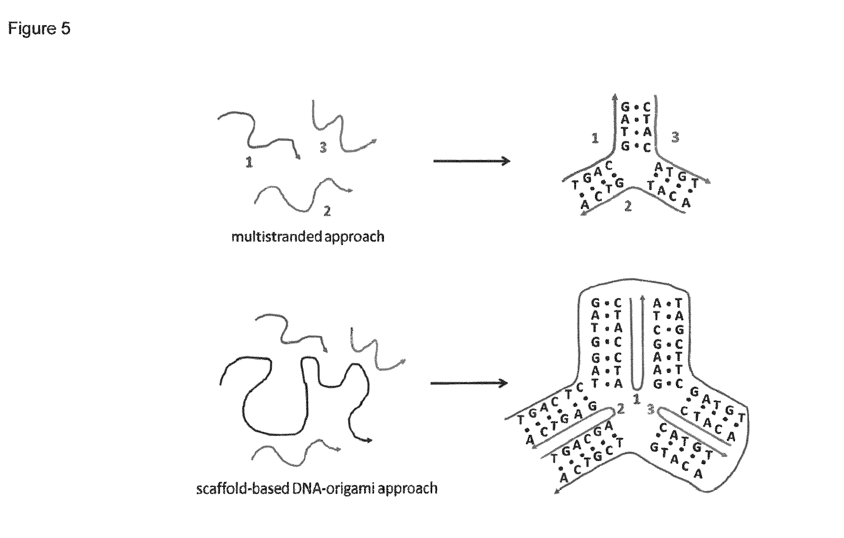

**Figure 5** shows two basic principles of self-assembly of single-stranded nucleic acids or nucleic acid binding molecules. **Figure a** demonstrates a multi-stranded approach wherein a plurality of staple strands is assembled to yield a predetermined structure. For the method of the invention, any of single-stranded molecules 1, 2 and 3 may represent the analyte. **Figure 5b** shows a scaffold-based approach wherein three staple strands and one longer scaffold strand assemble to yield a predetermined structure. In this aspect, the scaffold strand represents the analyte to be detected.

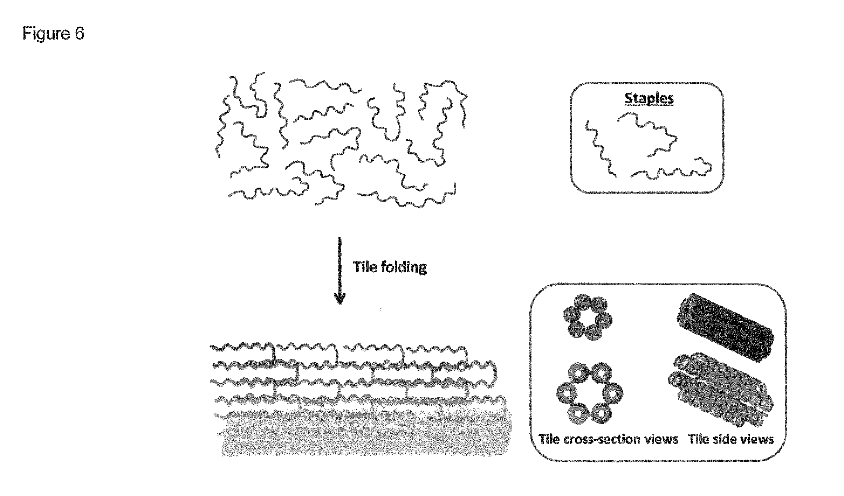

**Figure 6** is a detailed illustration of the multi-strand approach to generate a nanostructure of a plurality of staple strands. The staples act as tiles building up a so-called 6-helix bundle (6-HB).

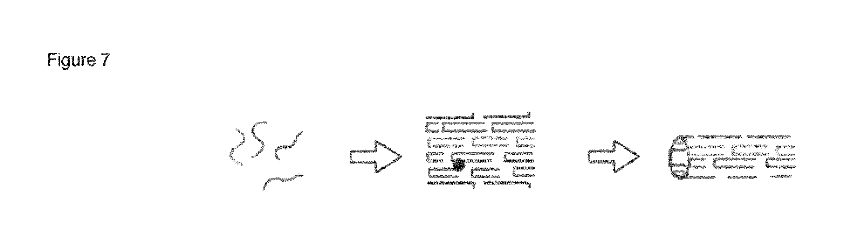

**Figure 7** shows the assembly of short staple strands in the absence of any scaffold.
**Figure 8** shows the stabilization of a nucleic acid nanostructure via click reactions. The plurality of staple strands used for self-assembling a scaffold strand comprises modified click reactive staples. Click reaction between corresponding click reactive groups provides a linkage at defined positions of the nanostructure. The resulting modified nanostructure features increased stability as compared to the unmodified nanostructure.
**Figure 9** is a schematic representation of a method for increasing stability of a nucleic acid nanostructure using staples containing an azide and an alkyne at the same staple ends. After folding and click reaction, each modified staple forms a closed ring annealed to the next ring formed by an adjacent modified staple.
**Figure 10** is a schematic representation of nucleic acid tile folding using staples containing an azide and an alkyne at the same staple end.
**Figure 11** illustrates click reactions occurring between the terminal azide and the terminal alkyne of a staple strand. As can be seen, the staples cyclize forcing two staples to form a two-ring chain. Under denaturing conditions the originally formed nanostructure is dissolved. An analysis using gel electrophoresis shows that the two-ring chain can be detected as a characteristic fragment.
**Figure 12** shows denaturing PAGE electrophoresis of nucleic acid tiles before and after cyclization via click chemistry with the formation of a two-ring chain.
**Figure 13** shows the formation of two distinct two-ring chains.
**Figure 14** shows the formation of one five-ring chain which can be detected using gel electrophoresis.
**Figure 15** illustrates a click reaction performed on a nanostructure formed from a mixture of staple strands including an elongated staple (green). The reactive groups of this elongated green staple are not pre-organized in the folded nanostructure and thus do not react with each other in a selective manner during the click reaction. Moreover, these groups (or only one of them) can be blocked by an extra molecule containing one of the complementary reactive groups which is added during the click reaction. This extra molecule is called competitor azide or competitor alkyne.
**Figure 16** shows the click reaction occurring on the same nanostructure. Due to the presence of a competitor, no ring is formed on the green staple as a result of the click reaction. Other click modified staples however are combined to form two-ring chains. Under denaturing conditions, the originally formed nanostructure is dissolved and two-ring chains can be detected as characteristic fragments.
**Figure 17** shows the formation of a similar nanostructure as in Figure 15, the difference being that an analyte (target) is now integrated into the nanostructure. The elongated green staple hybridizes to the target which results in a pre-organization of the reactive groups on the elongated green staple. In the presence of the target, the reactive groups come into close proximity of each other.
**Figure 18** illustrates the click reaction using the nanostructure of Figure 17. Due to the pre-organization of the reactive groups of the elongated green staple, the reactive groups promptly react forming a closed strand which sticks together with the other two-ring chains leading to detectable five-ring chain.
**Figure 19** illustrates the spatial re-organization of a nanostructure upon addition of a monovalent sodium salt.

### Examples

### Example 1: Detection of an analyte in a sample

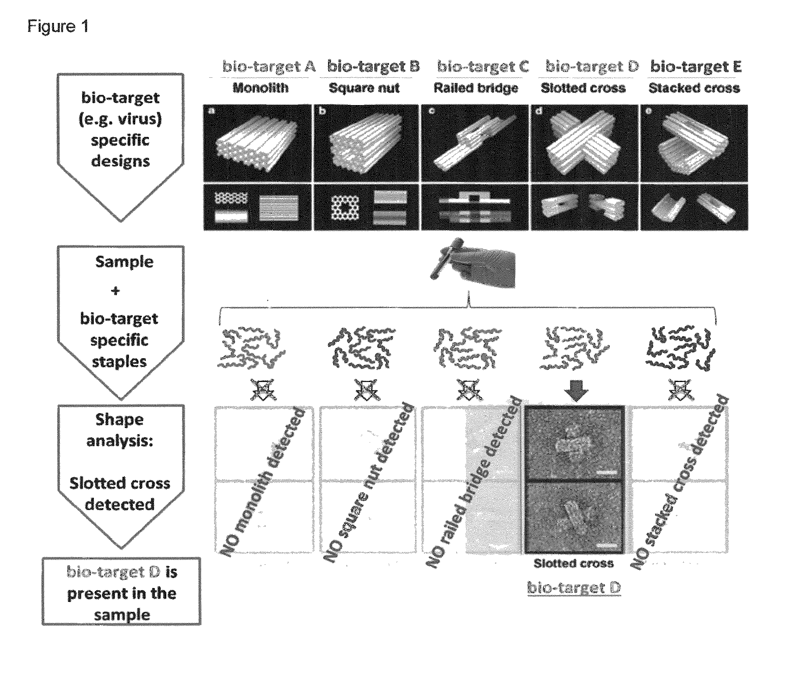

Specific staples are mixed with the sample in which the analyte (biotarget) has to be detected. If the analyte is present in the sample, then the staples organize themselves (hybridization step) in such a way to form a pre-defined shape, the characteristic nanostructure as illustrated in Figure 1. In the present example, staple strands specific for the different biotargets A-E are contacted with the sample. In the presence of biotarget A, self-assembly would result in the formation of a monolith structure. In the presence of biotarget B, self-assembly of the specific staple strands and the analyte would result in a square knot. Biotarget C would self-assemble with the staple strands to form a railed bridge. Biotarget D and the respective staples would self-assemble to form a slotted cross, and biotarget E would self-assemble together with the specific staples to yield a stacked cross. When the corresponding staples for the formation of all five shapes are brought into contact with the sample, a subsequent microscopic analysis demonstrates that only a slotted cross has formed. The formation of this particular nanostructure indicates the presence of biotarget D in the sample.

### Example 2: Detection of single nucleotide polymorphisms (SNPs) using a bar code reader

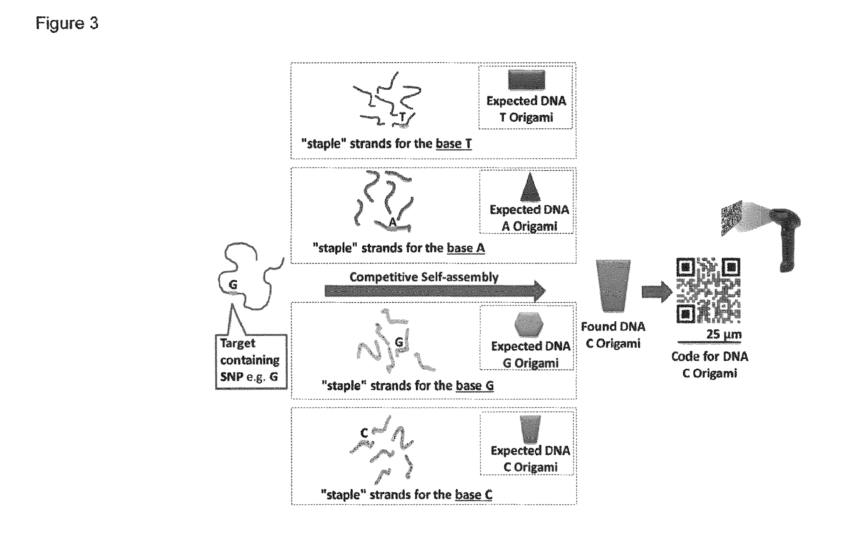

On a solid support, four different staple strands are immobilized. Each of the staple strands is characteristic for a particular base A, G, T and C. The various staple strands are immobilized on predetermined positions of the support. After that, a sample containing a nucleic acid wherein a polymorphism is to be detected is brought into contact with the modified solid support. The target nucleic acid binds to the immobilized staple strand which corresponds to the SNP contained in the target nucleic acid. In the presence of additional staple strands, a self-assembly of the target nucleic acid and the staple strands occurs and a characteristic nanostructure is formed. The use of appropriate folding conditions ensures that only the staple mixture containing the correct base (complementary to the SNP) will appropriately bind to the target and only one nanostructure will be generated as can be seen in Figure 3. The nanostructure can be determined either based on the shape of the nanostructure or using the position on the support where the nanostructure is formed.

### Example 3: Stabilization of a nanostructure

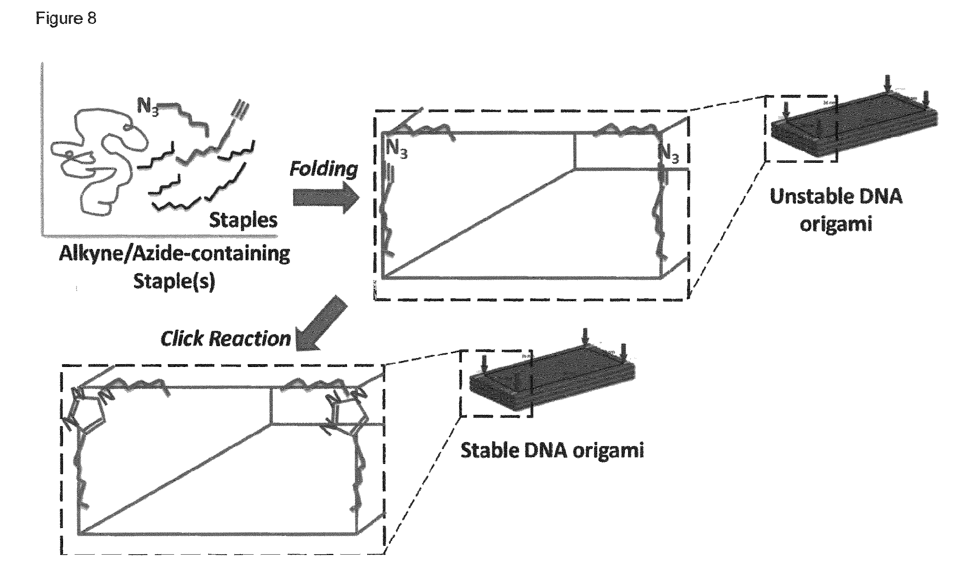

In order to increase the stability of a nucleic acid nanostructure, modified click reactive staples are included in the mixture of a plurality of staple strands. Click reactive groups are provided at those staple strands that occupy decisive positions in the folded nanostructure. When assembled to yield a predetermined nanostructure, these click reactive groups come in close proximity to each other as can be seen in Figure 8. In this way, the two functional groups can react with each other in a click reaction leading to a more stable configuration.

### Example 4: Click modified nanostructure

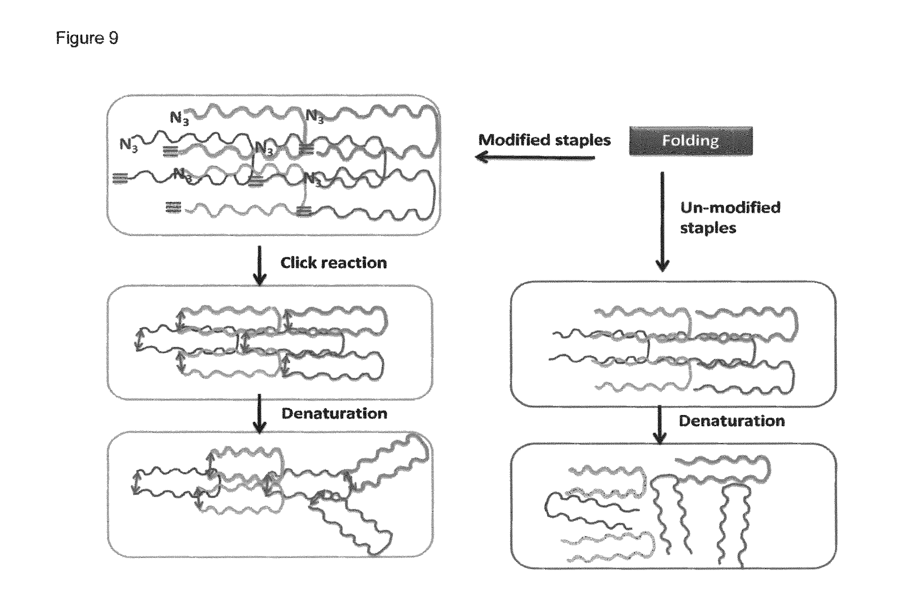

A plurality of staple strands is provided, some of which include reactive groups at both ends of the strand. During the hybridization (folding), each staple winds around its complementary staples many times (depending on the size of the staple) forming the double-helix typical for a DNA. If at least two of these staples are cyclized (covalent bond between the ends of each staple) while they are hybridized, then they will form a two-ring chain. If several of these staples cyclize at the same moment, then a 2D-chain is formed keeping compact together and thus stabilizing the nanostructure as can be seen in Figure 9. After folding and click reaction, each modified staple forms a closed ring annealed to the next ring formed by an adjacent modified staple. Stability tests confirm the enhanced stability of the structure in comparison with unmodified DNA origami.

### Example 5: Detection of characteristic fragments of a nucleic acid nanostructure

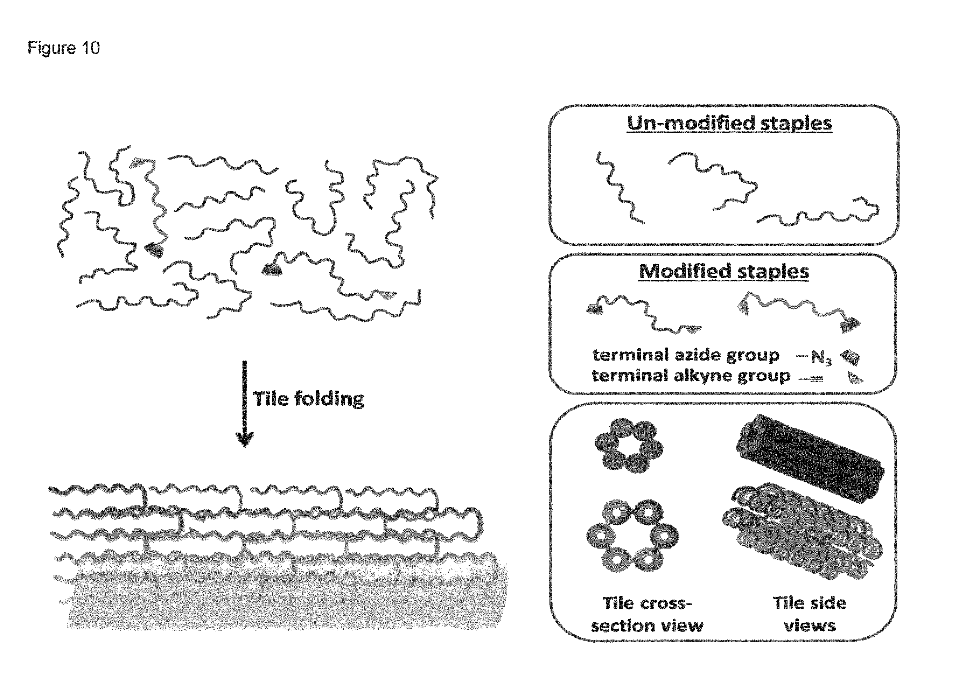

Experimentally, two modified staples (shown in green and in red in Figure 10) have been initially included in a staple mixture designed to obtain a predetermined nanostructure. Self-assembly resulting in the formation of this nanostructure can be effected using standard protocols. The procedure is illustrated in Figure 10.

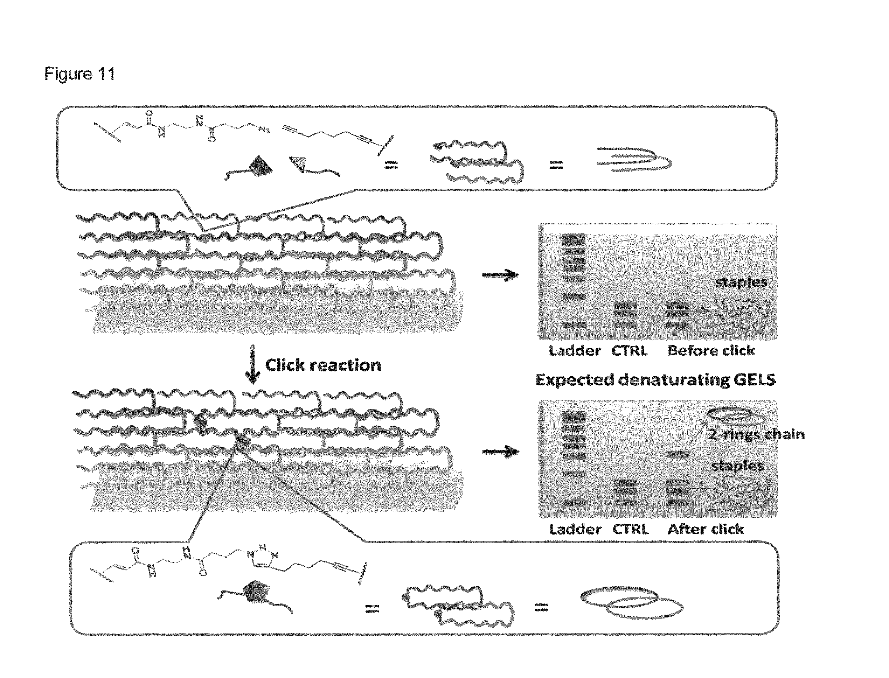

Subsequently, the terminal pre-organized modifications have been allowed to react, in this case via click chemistry by the addition of a Cu(I) source as shown in Figure 11. The reaction is selective and very efficient since the reactive groups face one another. Click reaction occurring between the terminal azide and the terminal alkyne of each staple results in cyclization forcing the two staples to form a two-ring chain.

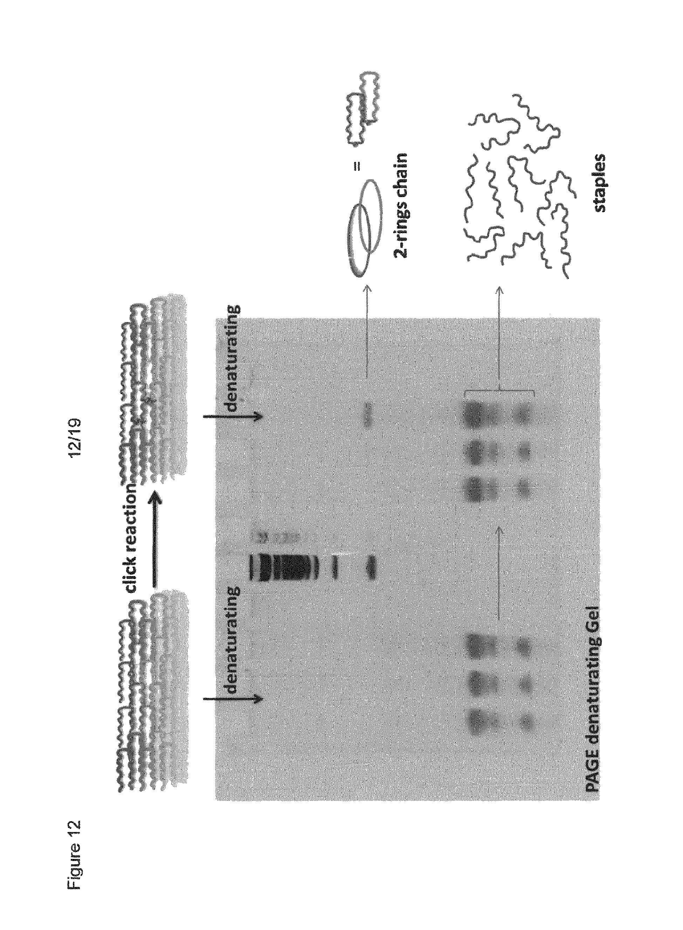

The expected result of denaturing gel electrophoresis is reported on the right side of Figure 11 for both unreacted nanostructure (first gel on the top: the staples are still open thus having about the same mass and length as all other staples) and reacted nanostructure (gel on the bottom of Figure 11: the staples are closed and fixed in a two-ring chain). This two-ring chain has a larger mass and larger length than the unreacted or the unmodified staples in the mixture. Thus, it can be easily detected as a new band in the gel. As shown in Figure 12, the gel electrophoresis (denaturing page) indeed shows the expected formation of a new band at the size corresponding to two staples running together in the gel. The clear observation of the new band obtained after click chemistry due to the formation of a two-ring chain is a strong evidence of the efficacy of this new approach to stabilize a nucleic acid nanostructure and also paves the way for the design of a new diagnostic effort based on the selective formation of those rings.

### Example 6: Detection of a nucleic acid analyte using click modified staples

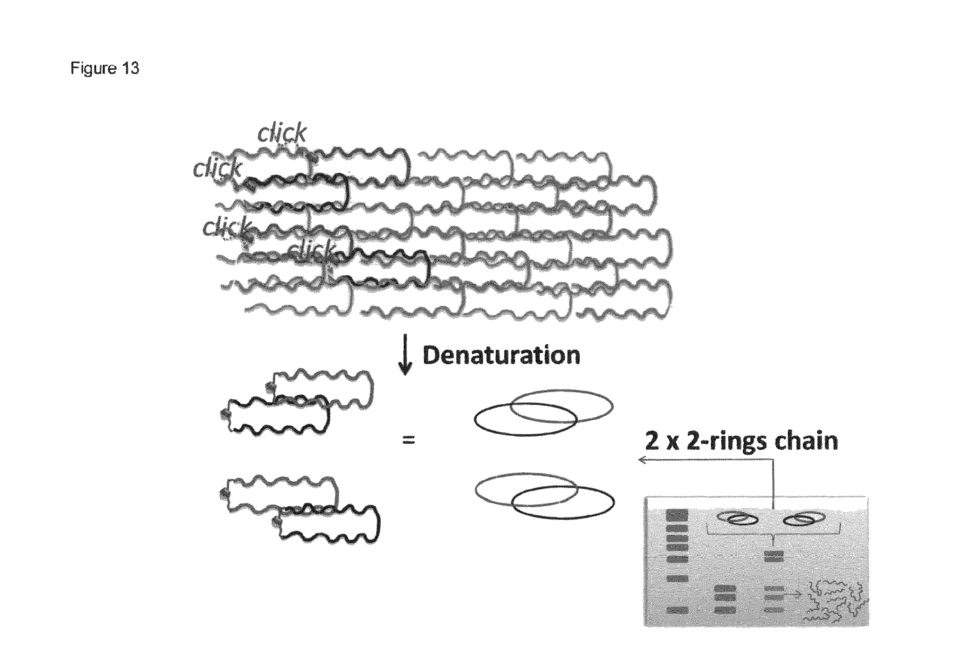

The formation of ring chains can be accurately and easily designed introducing modified staples at defined positions of the nanostructure. In Figure 13, for example, four modified staples are introduced into the staple mixture. After folding, the reactive groups present at the ends of each modified staple are pre-organized in a way to face each other. In this case, after the click reaction, two distinct two-ring chains are formed (Figure 13).

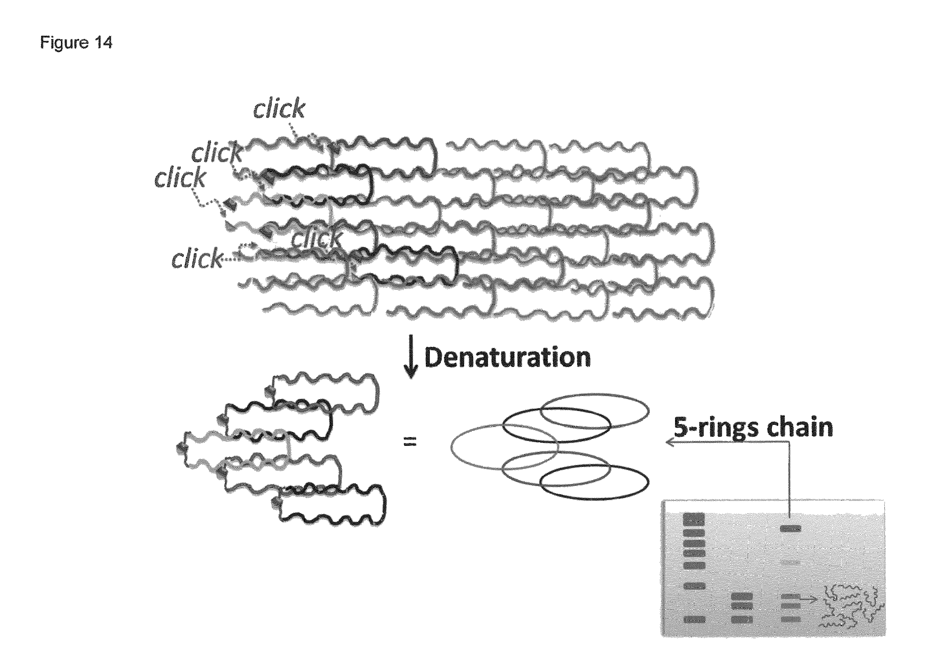

To connect these two two-ring chains, it is necessary to include another modified staple (shown in green in Figure 14) in the mixture before folding the nanostructure. The following click reaction ensures now the cyclization of the green staple as well and thus the formation of a five-ring chain as shown in Figure 14. This five-ring chain can be easily distinguished from the two-ring chain due to its larger size and higher weight. It appears in the denaturing gel as a new band.

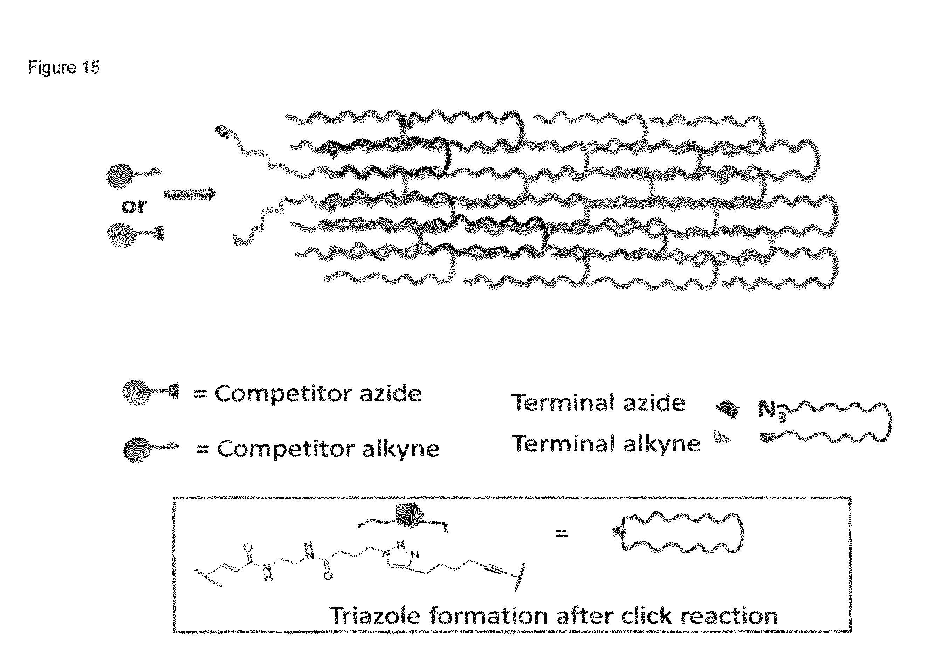

A diagnostic setup has been designed based on this principle. The fifth staple (green staple) has been designed in a way to have elongations (this staple is longer than needed for the formation of a DNA nanostructure). The elongated green staple is modified with the terminal reactive groups for the staple cyclization as well. The functional groups are a terminal azide and a terminal alkyne for subsequent click reaction in this example. However, in principle, any combination of two functional groups that are capable to react with each other forming a covalent linkage would be suitable as well. Click reaction resulting in ring formation is illustrated in Figure 15.

The reactive groups of the elongated green staples are not pre-organized in the folded DNA tile and thus will hardly react with each other in a selective manner during the click reaction. Moreover, these groups (or only one of them) can be blocked by an extra molecule containing one of the complementary reactive groups and added during the click reaction. This extra molecule is called *"competitor azide"* or *"competitor alkyne"* (cf. Figure 15).

The competitor will react faster with the terminal reactive group of the non-pre-organized green staple due to its small molecule nature and eventually its concentration. Anyway, the competitor will not react with the preorganized reactive groups of the modified staples (red and black staples) inside the folded nanostructure due to the steric hindrance of the effective groups inside the nanostructure. Moreover, the local concentration of the pre-organized reactive groups of the same staple can be considerably high since they face one another during the click reaction.

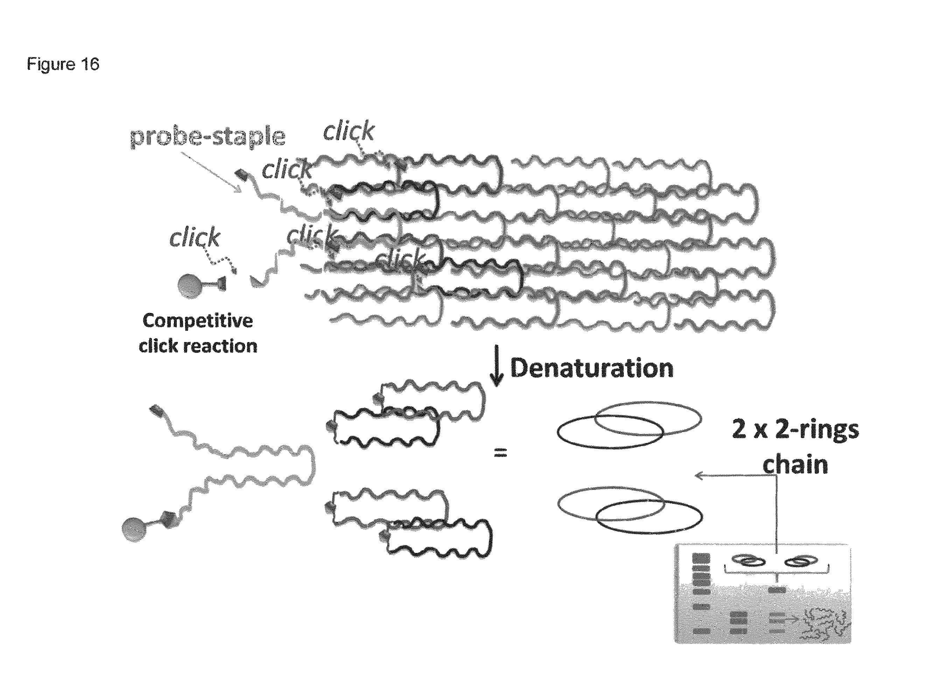

As a result of this click reaction in the presence of a competitor, two two-ring chains are formed and detected e.g. in the denaturing gel electrophoresis (Figure 16).

Similar to the other modified staples inside the nanostructure, if both reactive groups of the elongated green staple are pre-organized, they will react faster than with the competitor ensuring the cyclization of this staple as well and thus the formation of an easily detectable five-ring chain.

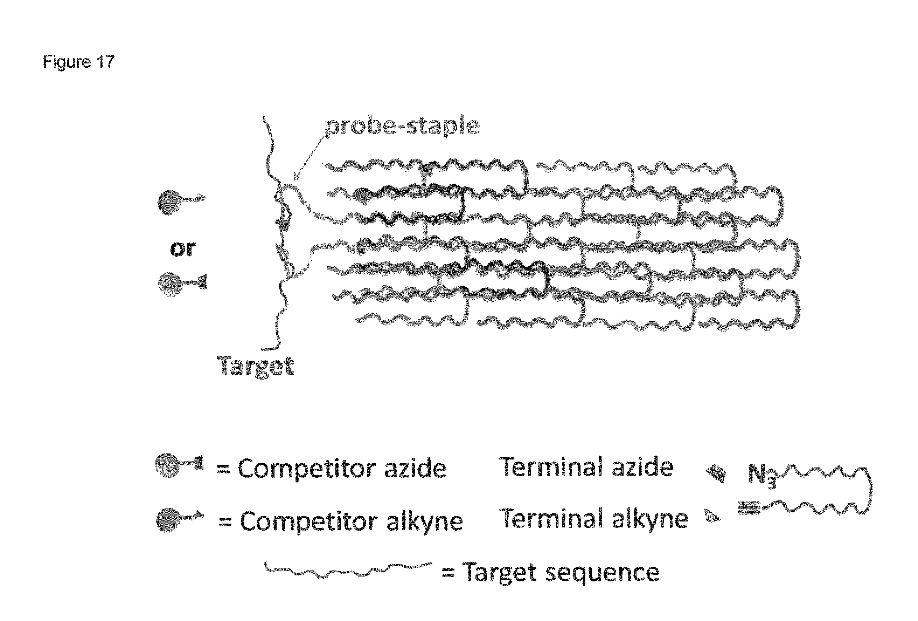

We designed the elongated green staple using a sequence which is complementary to a target sequence (e.g. an analyte which may for example be viral DNA or viral RNA, a gene, transgene etc.). Similar to the in-situ hybridization principle, the folded nanostructure will recognize and hybridize with its target through the elongations of the green staple. If such a target (shown in red in Figure 17) is present in the mixture to be analyzed, then the elongated green staple will specifically anneal with it. As a result of this hybridization, the reactive groups of the elongated green staple are now preorganized and are ready to react much faster with one another when the click reaction is started (addition of click reagents) even in the presence of a competitor as described herein above for the modified staples (red and black) located inside the nanostructure.

Obviously the elongated green staple could be designed in a way to be cyclized via enzymatic ligation or other reaction binding together the terminal ends of this strand.

The hybridization of the nanostructure with the target can be afforded after the formation of the nanostructure or even during the folding process which normally includes heating and slow cooling of the staple mixture exactly as any other hybridization protocol.

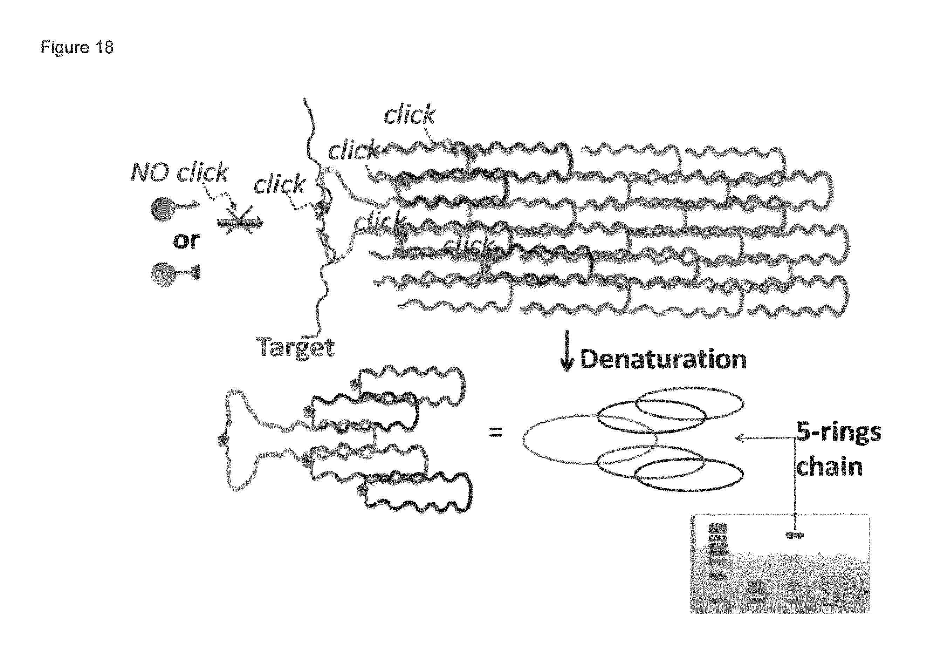

The click reaction in presence of the specific target and of a competitor leads to the cyclization of the elongated green staple, which results in the formation of an easily detectable five-ring chain as depicted in Figure 18.

**The following items represent preferred embodiments of the invention:**
1. A method for detecting an analyte in a sample, comprising
   (i) contacting the sample with a plurality of staple strands
   (ii) providing conditions suitable to induce self-assembly of the staple strands and the analyte to yield a nanostructure, and
   (iii) determining if a predetermined nanostructure is formed, wherein the formation of the predetermined nanostructure indicates the presence of the analyte in the sample.
2. The method of item 1, wherein step (iii) comprises
   (a) providing conditions wherein a nanostructure resulting from step (ii) is at least partially dissolved to yield fragments, and
   (b) detecting fragments of the predetermined nanostructure.
3. The method of item 1 or 2, wherein in step (ii) staple strands and analyte are self-assembled on a solid support.
4. The method of item 3, wherein the self-assembled nanostructures are spatially self-organized on the solid support.
5. The method of item 4, wherein the self-organization of the self-assembled nanostructures is re-directed by the addition of salts.
6. The method of item 4 or 5, wherein the position of the self-organized nanostructures is fixed by the addition of specific reagents or other entities.
7. The method of any one of items 3-6, wherein in step (i) at least one staple strand is immobilized on a solid support or comprises a linker group for immobilizing the staple strand to a solid support, preferably at a predetermined position on the support.
8. The method of any one of items 3-7, wherein step (iii) comprises detecting the presence or absence of the nanostructure of the support, preferably the presence or absence of the nanostructure at a predetermined position on the support.
9. The method of item 7 or 8, wherein in step (i) a plurality of staple strands each specific for an analyte is immobilized on the solid support or comprises a linker group for immobilizing the staple strand to a solid support, preferably at a predetermined position on the support.
10. The method of any one of the preceding items, wherein at least one of the staple strands comprises a functional group, such as a marker group, a linker group (e.g. for attaching to a solid support) or a handle group (for introducing a functional group).
11. The method of any one of the preceding items, wherein at least one staple strand comprises a first functional group and at least one staple strand comprises a second functional group, capable of reacting with the first functional group.
12. The method of item 11, wherein in the presence of the analyte, a nanostructure is formed in step (ii), wherein the first and the second functional groups are preorganized to come in close vicinity, and wherein the first and the second functional groups are reacted with each other to form a linkage.
13. The method of item 11 or 12, wherein the first and the second functional groups are present on different staple strands.
14. The method of item 11 or 12, wherein at least one staple strand comprises both a first and a second functional group
15. The method of any one of items 11 to 14, wherein the first and the second functional groups are Click functional groups capable to react with each other to form a 1,2,3 - triazole linkage.
16. The method of any one of items 11 to 15, wherein said linkage stabilizes the nanostructure.
17. The method of any one of items 11 to 16, wherein linkage of the first and the second functional groups forms a ring structure.
18. The method of any one of items 1 to 17, wherein at least one staple strand comprises a detectable moiety, preferably a fluorescent label.
19. The method of any one of the preceding items, wherein the analyte is selected from nucleic acids and nucleic acid-binding molecules.
20. The method of any one of the preceding items, wherein the nanostructure and/or fragments thereof are detected using mass spectroscopy, optical methods, microscopic methods such as electron microscopy, atomic force microscopy and fluorescence microscopy, and/or gel electroporesis.
21. The method of any one of the preceding items , for detecting a nucleic acid, nucleic acid analogue, SNP, an organism and/or a gene associated to it.
22. A kit for detecting an analyte, comprising
   (i) a plurality of staple strands, and optionally
   (ii) a solid support
   wherein the staple strands are capable to self-assemble with the analyte to yield a predetermined nanostructure.
23. The kit of item 22, wherein at least one of the staple strands is immobilized on the solid support or comprises a linker group for immobilizing the staple strand on the solid support, preferably at a predetermined position on the solid support.
24. A device for detecting an analyte in a sample, comprising
   (i) a sample loading unit,
   (ii) a kit of item 22 or 23,
   (iii) means for processing the sample, and
   (iv) means for determining if a predetermined nanostructure is formed.
25. A method for providing a stabilized nucleic acid nanostructure, comprising the steps
   (i) providing a plurality of staple strands, wherein at least one strand comprises a first functional group and at least one strand comprises a second functional group, capable of reacting with the first functional group,
   (ii) providing conditions suitable to induce self-assembly of the staple strands to yield a predetermined nanostructure, wherein the first functional group and the second functional group are preorganized to come in close vicinity, and
   (iii) reacting the first and the second functional groups to yield a linkage.
26. A kit for providing a stabilized nucleic acid nanostructure comprising a plurality of staple strands, wherein at least one strand comprises a first functional group and at least one strand comprises a second functional group, capable of reacting with the first functional group to form a linkage,
   wherein the staple strands are capable to self-assemble, optionally together with a target strand, to yield a predetermined nanostructure.
27. The kit of item 26, wherein at least one of the staple strands is immobilized on the solid support or comprises a linker group for immobilizing the staple strand on the solid support.
28. A stabilized nanostructure, comprising a plurality of assembled staple strands and at least one covalent linkage including a 1,2,3-triazole moiety.

## Claims

1. A method for detecting an analyte in a sample, comprising
(i) contacting the sample with a plurality of staple strands
(ii) providing conditions suitable to induce self-assembly of the staple strands and the analyte to yield a nanostructure, and
(iii) determining if a predetermined nanostructure is formed,
wherein the formation of the predetermined nanostructure indicates the presence of the analyte in the sample.

2. The method of claim 1, wherein step (iii) comprises
(a) providing conditions wherein a nanostructure resulting from step (ii) is at least partially dissolved to yield fragments, and
(b) detecting fragments of the predetermined nanostructure.

3. The method of claim 1 or 2, wherein in step (ii) staple strands and analyte are self-assembled on a solid support, preferably wherein the self-assembled nanostructures are spatially self-organized on the solid support.

4. The method of claim 3, wherein the self-organization of the self-assembled nanostructures is re-directed by the addition of salts, and optionally wherein the position of the self-organized nanostructures is fixed by the addition of specific reagents or other entities.

5. The method of any one of claims 3-4, wherein in step (i) at least one staple strand is immobilized on a solid support or comprises a linker group for immobilizing the staple strand to a solid support, preferably at a predetermined position on the support.

6. The method of any one of claims 2-5, wherein step (iii) comprises detecting the presence or absence of the nanostructure on the support, preferably the presence or absence of the nanostructure at a predetermined position on the support, in particular wherein in step (i) a plurality of staple strands each specific for an analyte is immobilized on the solid support or comprises a linker group for immobilizing the staple strand to a solid support, preferably at a predetermined position on the support.

7. The method of any one of the preceding claims, wherein at least one of the staple strands comprises a functional group, preferably wherein at least one staple strand comprises a first functional group and at least one staple strand comprises a second functional group, capable of reacting with the first functional group.

8. The method of claim 7, wherein in the presence of the analyte, a nanostructure is formed in step (ii), wherein the first and the second functional groups are preorganized to come in close vicinity, and wherein the first and the second functional groups are reacted with each other to form a linkage, preferably wherein the first and the second functional groups are present on different staple strands, and/or wherein at least one staple strand comprises both a first and a second functional group.

9. The method of any one of claims 7 to 8, wherein the first and the second functional groups are Click functional groups capable to react with each other to form a 1,2,3-triazole linkage.

10. The method of any one of claims 7 to 9, wherein said linkage stabilizes the nanostructure, and/or wherein linkage of the first and the second functional groups forms a ring structure.

11. The method of any one of claims 1 to 10, wherein at least one staple strand comprises a detectable moiety, preferably a fluorescent label.

12. The method of any one of the preceding claims, wherein the analyte is selected from nucleic acids and nucleic acid-binding molecules.

13. The method of any one of the preceding claims, wherein the nanostructure and/or fragments thereof are detected using mass spectroscopy, optical methods, microscopic methods such as electron microscopy, atomic force microscopy and fluorescence microscopy, and/or gel electroporesis.

14. A kit for detecting an analyte, comprising
(i) a plurality of staple strands, and optionally
(ii) a solid support
wherein the staple strands are capable to self-assemble with the analyte to yield a predetermined nanostructure, preferably wherein at least one of the staple strands is immobilized on the solid support or comprises a linker group for immobilizing the staple strand on the solid support, preferably at a predetermined position on the solid support.

15. A device for detecting an analyte in a sample, comprising
(i) a sample loading unit,
(ii) a kit of claim 14,
(iii) means for processing the sample, and
(iv) means for determining if a predetermined nanostructure is formed.

16. A method for providing a stabilized nucleic acid nanostructure, comprising the steps
(i) providing a plurality of staple strands, wherein at least one strand comprises a first functional group and at least one strand comprises a second functional group, capable of reacting with the first functional group,
(ii) providing conditions suitable to induce self-assembly of the staple strands to yield a predetermined nanostructure, wherein the first functional group and the second functional group are preorganized to come in close vicinity, and
(iii) reacting the first and the second functional groups to yield a linkage.

17. A kit for providing a stabilized nucleic acid nanostructure comprising a plurality of staple strands, wherein at least one strand comprises a first functional group and at least one strand comprises a second functional group, capable of reacting with the first functional group to form a linkage, wherein the staple strands are capable to self-assemble, optionally together with a target strand, to yield a predetermined nanostructure.

18. A stabilized nanostructure, comprising a plurality of assembled staple strands and at least one covalent linkage including a 1,2,3-triazole moiety.
